# EUROPEAN PATENT SPECIFICATION

(11) **EP 0 355 199 B1**
(45) Date of publication and mention of the grant of the patent: **21.04.1993**
(21) Application number: 88116413.1
(22) Date of filing: 04.10.1988
(51) Int. Cl.: A61M 1/00

(54) **Aspirator for the collection of bodily fluids**
Aspirator zur Aufnahme von Körperflüssigkeiten
Aspirateur pour collecter des liquides corporels

(30) Priority: 22.06.1988 US 209852; 22.06.1988 US 210076
(43) Date of publication of application: 28.02.1990
(73) Proprietor: ROSENBLATT/IMA INVENTION ENTERPRISES (a joint venture formed under the laws of the State of California), Beverly Hills, California (US)
(72) Inventor: Rosenblatt, Richard, Beverly Hills, California 90212 (US)
(74) Representative: Baillie, Iain Cameron

(56) References cited:
- US-A- 3 084 691
- US-A- 3 343 422
- US-A- 4 392 860
- US-A- 4 397 643
- US-A- 4 775 366

## Description

### BACKGROUND OF THE INVENTION

### 1. FIELD OF THE INVENTION

The present invention relates to aspirators. More particularly, the present invention relates to an aspirator for removing bodily fluid from a body cavity, such as mucus from the mouth and throat, through oral suction by another person.

### 2. DESCRIPTION OF THE PRIOR ART

It is often necessary to remove bodily fluids, such as mucus and other matter, from the throat of a patient, especially in the case of newborn infants. Vacuum operated collection devices for collecting such bodily fluids are known in prior art.

Such collection devices generally include a container having a screw-on or snap-on cap that provides a fluid-tight closure, and a pair of tubes connected to nipples protruding from the cap in fluid communication with the interior of the container and with each other. In use, one of the tubes is connected to a source of vacuum or a suction force, for example, a mouthpiece for providing suction by mouth, or to another conventional source of hospital vacuum. The other tube may be inserted into the throat or other body cavity of the patient to permit withdrawal of fluid from the bodily cavity, and its collection in the container, in response to suction.

An example of this type of fluid collection device is found in U.S. Patent No. 4,317,525, issued to Schuessler et al. and in U.S. Patent 4,397,643 to Rygiel.
Such devices, however, allow air from the patient's body cavity to enter the suction tube, where bacteria or germs in it can contaminate and infect either a person who is sucking, or a hospital suction system.

Another such fluid collection system is disclosed in U.S. Patent No. 3,084,691, to Stoner. Stoner includes a foot-operated bellows pump for creating suction in a collection chamber having two nipples and mating tubes attached thereto, with one of the nozzles being used to suck bodily fluids from a body cavity. The apparatus in Stoner is relatively large, bulky, complex and expensive. In addition, it does not provide any indication of the amount of resistance to the sucking, which provides important feedback to a person using the device, who can responsively apply only the suction necessary to remove the subject liquids. Finally, Stoner too allows communication of the air to the nurse from the patient through the pump.

While the prior art discloses bodily fluid collection devices relying on suction, such devices allow communication of air, and other gas from the patient through the device, and in the instance of a manually operated device, into the nurse or other health care provider. Although such devices do not normally allow liquid from the patient to enter into the suction system, they do allow air or other gas to enter into the suction tube and the source of vacuum, thereby increasing the danger of further spreading of communicable diseases.

Therefore, there is a significant need for a bodily fluid collection device that isolates both the liquid and the gas fluids extracted from a patient by the health care provider, through a suction apparatus.

### SUMMARY OF THE INVENTION

Accordingly, it is an object of the present invention to provide a bodily fluid collection device that completely isolates gaseous and liquid fluids collected from the patient from the source of suction, which may conveniently be a person, such as a nurse.

It is a further object of the present invention to provide a bodily fluid collection device that is disposable.

It is a further object of the present invention to provide a bodily fluid collection device that is relatively inexpensive to manufacture.

It is a further object of the present invention to provide a bodily fluid collection device that is easy to open, thereby making the contents readily available for analysis.

It is a further object of the present invention to provide a disposable bodily fluid collection device that is sterile prior to use.

It is another object of the present invention to provide a bodily fluid collection device with several enhancements to aid in the efficiency and safety of operation, such as a moisture trap to prevent fluid from accidentally being sucked in by a person using the present invention to treat a patient, a pressure relief valve to depressurize the container as the bellows expand to its equilibrium state to thereby prevent air from being forced back through the patient tube and into the patient's stomach, an injection port by which the mucus may be withdrawn by a laboratory technician through use of a syringe so that the cap need not be opened and the risk of infection through direct contact with the mucus eliminated, and a clip to occlude air or liquid flow through the patient tube after the sucking operation has been completed.

It is a further object of the present invention to also enable the invention to be used in battlefield conditions to suck mucus and other fluids out of the mouths of wounded soldiers. By fitting the patient tube with an interchangeable Yankower tip, the same aspirator of the present invention can be used over and over again by replacing the Yankower tip when a new wounded soldier is worked on.

Accordingly, there is provided a container having a bottom wall and a sidewall, a top having two apertures therein, or the equivalent, such as two protruding nipples, removably attached to the container, a patient tube inserted through one of the apertures for insertion into a patient's body cavity, and a suction tube attached through the other aperture and having its remote end attached to a source of suction, which may be a person, or another conventional source of vacuum, and a means for transmitting a partial vacuum throughout the container and the patient tube without allowing fluid, that is, either gas or liquid, communication between the two tubes.

The vacuum transmitting means comprises a bellows that is expanded in its equilibrium or relaxed state, and that contracts in response to negative pressure, that is, in response to sucking on the suction tube, the suction tube being operatively connected to the bellows. In the preferred embodiment, the bellows is contained entirely within the container.

The invention may also include means for releasing air from the container during the relaxation cycle of the bellows, that is, means for permitting air to be exhausted from the container while the bellows expand without having the displaced air exhausted through the patient tube. This air releasing means may further comprise an aperture (6 as illustrated in Figure 6) in the sidewall of the container, or in the sucking tube (aperture 8 as illustrated in Figure 6) which is covered (for example by the operator) during sucking, and uncovered while the bellows is being restored to its equilibrium, that is, fully opened position. Alternatively, this air releasing means may be a spring loaded valve fit inside an aperture in the sidewall or top of the container. The valve is normally closed so no air can enter the container or escape from the container through this aperture. When a push button or similar release mechanism connected to the valve is activated, the valve opens to let air escape from the chamber, thereby depressurizing the entire container so the bellows can expand to its equilibrium position much more rapidly without the possibility of air or liquid being pushed up the patient tube and back into the patient's stomach.

Alternatively, the air releasing means may be automatic, and may include, for example, a ball valve seat disposed in the patient tube, preferably in the end of the patient tube that is contained within the container, the ball valve being forced closed during the relaxation or expansion cycle of the bellows, and drawn open during sucking, and a flap valve in the container, with the flap vale being naturally in the closed position during expansion of the bellows into its equilibrium position. Such air releasing means is not, however, necessary for proper and efficacious operation of the invention and may be too expensive for mass production of disposable aspirators.

Another feature of the invention comprises an upstanding partition within the container, which is contiguous with the sidewall of the container along two lines and along the bottom wall, forming two chambers, which are a liquid collection chamber and a vacuum transmitting chamber, wherein said chambers are in gaseous communication, but not in liquid communication, because the upstanding partition does not extend to the top of the container. Plastic is a preferred material for making the container, the top and the nipple in the top for attaching the suction tube to the container. A nipple in the top of the container may also be used for attaching of the patient tube.

In a preferred embodiment, the container is cylindrical. The upstanding partition may be either straight, or arcuate. An arcuate upstanding partition is preferred because its use allows more efficient use of the space available within the container.

Another enhancement for safer utilization of the present invention is to provide a moisture trap inside the suction tube which acts as a moisture block. The moisture trap is a filter which permits air to pass when it is dry but if moisture is present, the filter swells up and occludes the passageway, preventing air from passing. This is a valuable safety enhancement in the event the bellows or other sealing arrangement should break and moisture from the sucked contents of the baby,s stomach enter the suction system. By providing this occlusion, the possibility of the the person working the apparatus sucking in such material or sucking in contaminated air is significantly reduced.

A further enhancement for more efficient operation of the present invention is the inclusion of an injection port located in the area where mucus is collected, for example in the cap immediately above the mucus trap area of the present invention aspirator. The injection portion includes an opening having a structural support member which supports a diaphragm. After the mucus is collected, instead of opening the cap to remove the mucus, the laboratory technician can place the needle of a syringe through the membrane and draw out the mucus into the syringe, thereby eliminating the possibility of coming in direct contact with contaminated mucus.

An additional enhancement is the inclusion of a clip on the patient tube to occlude flow of air or liquid after the suction process is completed, thereby further assuring that no mucus can flow out of the suction tube.

An additional improvement in the present invention is to include a Yankower suction tip at the end of the patient tube so that the apparatus may be switched from patient to patient by replacement of the Yankower tip. This is especially useful in battlefield conditions where the present invention aspirator may be used to remove mucus and other debris from the mouth of a wounded soldier. Due to the extreme conditions of battle, it is easier to maintain the same apparatus and merely switch suction tips when treating many different wounded soldiers.

Other alternative embodiments utilize two separate containers, one for containing the bellows, and the other for collecting the bodily fluid, and a means for conducting gas from one container to another. Such means may include an externally disposed flexible tube connected to the top of each container, or an internal partition. In either case, the bodily fluid collection chamber is readily detachable from the vacuum generating chamber, for permitting easy access to the collected liquid, for medical testing.

Further novel features and other objects of the present invention will become apparent form the following detailed description, discussion, and the appended claims taken in conjunction with the drawings.

### BRIEF DESCRIPTION OF THE DRAWINGS

Referring particularly to the drawings for the purpose of illustration only and not limitation, there is illustrated:
FIGURE 1 is a perspective view of an aspirator according to the present invention.
FIGURE 2 is a section elevation of an aspirator according to the present invention, illustrating the bellows in their relaxed, or equilibrium position.
FIGURE 3 is a section of an aspirator according to the present invention, taken along lines 3-3 of FIGURE 2.
FIGURE 4 is a plan view of an aspirator according to the present invention, without the tubes attached to the top.
FIGURE 5 is a section of a aspirator according to the present invention, corresponding to FIGURE 3, but showing an aspirator having an arcuate internal upstanding partition.
FIGURE 6 is a partial cross-sectional elevation of an aspirator according to the present invention illustrating the bellows in their contracted, that is vacuum-creating state.
FIGURE 7 is an exploded side elevation, partially fragmentary, illustrating the cap, bellows, and suction tube fitting for the vacuum-creating portion of the apparatus.
FIGURE 8 is a plan view of an alternative embodiment of an aspirator according to the present invention in which separate circular containers are employed.
FIGURE 9 is a fragmentary elevation of the apparatus of FIGURE 8.
FIGURE 10 is a plan view of an alternative embodiment of an aspirator according to the present invention in which separate circular containers are employed, and are joined in gaseous communication by an external tube that penetrates the top of each container.
FIGURE 11 is a fragmentary elevational view of the apparatus of FIGURE 10.
FIGURE 12 is a fragmentary elevational of the suction tube according to the present invention, including the mouthpiece.
FIGURE 13 is an elevation of the patient tube adapted for use with the present invention, illustrating the uniform taper that lodges the patient tube into correct position within the aspirator, and the Murphy tip at the remote end for insertion into the body cavity to be drained.
FIGURE 14 is a perspective view of an aspirator according to the present invention, including several improvements for safer and more efficient operation.
FIGURE 15 is a section elevation of an aspirator according to the present invention with improvements shown in Figure 14, illustrating the bellows in their relaxed, or equilibrium position.
FIGURE 16 is an enlarged view of the cap of the aspirator with improvements as illustrated in FIGURE 15.
FIGURE 17 is an enlarged cross-sectional view of the cap of the aspirator, showing another alternative method by which the bellows cap can be attached to the cap of the aspirator.
FIGURE 18 is an enlarged cross-sectional view of the cap of the aspirator, showing a further alternative method by which the bellows cap can be attached to the cap of the aspirator.
FIGURE 19 is an enlarged cross-sectional view of the cap of the aspirator, showing an alternative embodiment of the bellows cap attached directly to the suction tube.
FIGURE 20 is a perspective view of a Yankower tip.
FIGURE 21 is a perspective view of an alternative embodiment of the aspirator as shown in FIGURE 14, with the internal partition wall removed.
FIGURE 22 is a section elevation of the alternative embodiment of the aspirator shown in FIGURE 21, with the internal partition wall removed.

### DETAILED DESCRIPTION OF THE PREFERRED EMBODIMENT

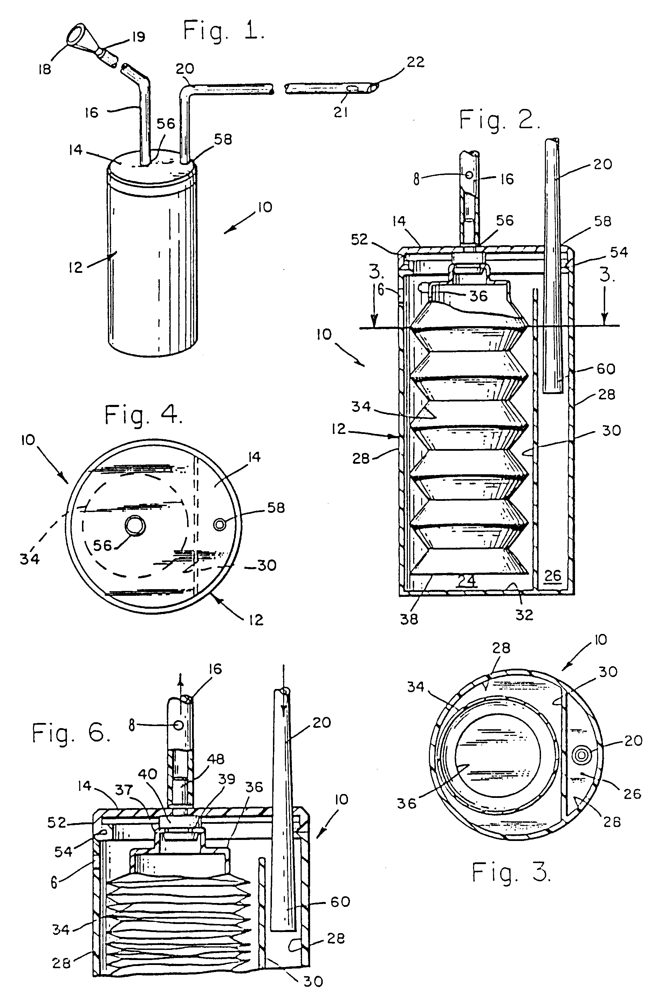

Referring to FIGURE 1, there is shown aspirator 10 having a cylindrical container 12, cap 14, suction tube 16, terminating in flared mouthpiece 18 which is a separate piece having nipple 19 which is inserted into suction tube 16, and patient tube 20, terminating in Murphy tip 22, all illustrated in perspective. Murphy tip 22 consists of cutting patient tube 20 at an oblique angle to present a more easily inserted surface, and providing aperture 21 adjacent to the tip, as is well-known in the art.

In the preferred embodiment illustrated in FIGURE 1, suction tube 16 and patient tube 20 are made of a flexible, rubber-like material, typically rubber or a synthetic substitute, such as polyvinyl chloride or other material well known in the art. Cylindrical container 12 is preferably made of a hard plastic material, by means of injection or blow-molding, but may be made of any rigid material, such as metal or hard rubber. Aspirator 10 is preferably disposable and is about 5 centimeters in outside diameter and about 10 centimeters long.

In use, aspirator 10 is hung from the user's neck by a cord, so that aspirator 10 is at about mid-chest level, and mouthpiece 18 is inserted into the user's mouth. Murphy tip 22 of patient tube 20 is inserted into the body cavity to be drained, and then the user sucks on flared mouthpiece 18, creating a partial vacuum that is communicated through patient tube 20 to the patient, by means that will be described in greater detail below, thereby drawing liquid and other fluid from the body cavity of the patient. When used as described, aspirator 10 leaves both of the user's hands free, to hold the patient and manipulate patient tube 20. That is, the use of the hands is not required to hold or otherwise manipulate aspirator 10 or mouthpiece 18, a great advantage in using the present invention. When used in this fashion, the preferred length of suction tube 16 is approximately 20 to 30 centimeters, and the preferred length of patient tube 20 is about 30 to 40 centimeters.

Referring to FIGURE 2, cylindrical container 12 comprises two internal chambers, vacuum creating chamber 24, and mucus trap 26, created by sidewall 28, and internal upstanding partition 30, which is integrally formed with sidewall 28 of cylindrical container 12 during molding, and is attached to bottom wall 32, as well as two lines of joinder with sidewall 28, as is best illustrated in FIGURE 3. Internal upstanding partition 30 rises upward through most of the height of cylindrical container 12, preferably rising a distance within the range of one-third to seven eighths of the length of the container, but does not reach cap 14, thereby creating a path for communication of air and other gaseous fluid between vacuum creating chamber 24 and mucus trap 26.

Vacuum creating chamber 24 contains bellows 34, attached to bellows cap 36 by being integrally formed therewith, or attached by a suitable adhesive. Bellows 34 is preferably made from silicon rubber, having a thickness of from about 1 to about 1.5 millimeters. Silicon rubber is a material well known in the art of medical supplies and is also employed in such well known household items as baby bottle nipples. Silicon rubber has an excellent memory, which consistently restores an article formed from it to its original shape, if it is deformed and then the deforming force is removed. Therefore, no spring or other elastic member is required in bellows 34. Bellows 34 is preferably formed in a single unitary piece, having a sealed bottom 38 formed of the same material, by blow molding.

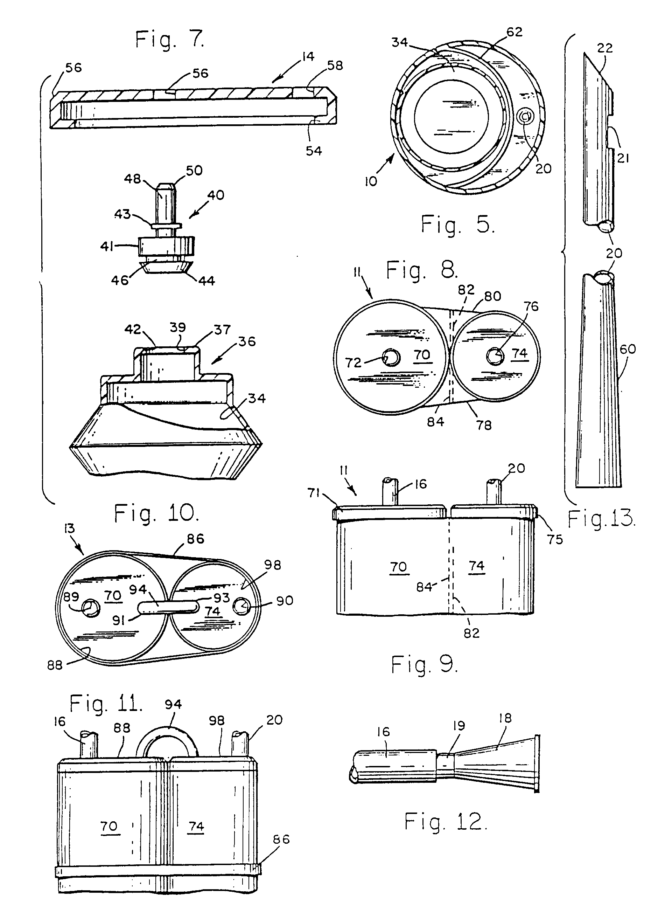

Referring to FIGURE 7, there is most clearly illustrated the relationship between bellows 34, bellows cap 36, bellows stopper 40, and cap 14. Bellows cap 36 may conveniently be formed from hard rubber or the like and attached to the top of bellows 34 with a conventional adhesive. The top of bellows cap 36 comprises aperture 42 for receiving bellows stopper 40, made of pliable rubber or the like, and having a tapered nose section 44, for easing penetration of aperture 42, slot 46, circumferentially disposed about the base of bellows stopper 40, adjacent to tapered nose section 44, for further easing penetration of aperture 42 by bellows stopper 40 into bellows cap 36, and in which top wall 37 of bellows cap 34 is seated when bellows stopper 40 is inserted through aperture 39 of bellows cap 36.

Attached to the top of bellows stopper 40 and integrally formed therewith is nipple 48, for allowing attachment of suction tube 16. Central orifice 50 penetrates the entire length of bellows stopper 40, allowing gaseous communication from suction tube 16 to the interior of bellows 34.

Cap 14 includes depending circumferential skirt portion 52, which terminates in inwardly projecting circumferential lip 54, and includes suction tube aperture 56, and patient tube aperture 58, in its top portion.

Suction tube aperture 56 is penetrated by nipple 48 of bellows stopper 40 until cap 14 is sandwiched between base section 41 and flange 43 of bellows stopper 40. Mouthpiece 18, suction tube 16, bellows stopper 40, and bellows 34 thereby provide a closed system with mouthpiece 18 providing the only means of entry and exit of gas or other fluid into or out of bellows 34.

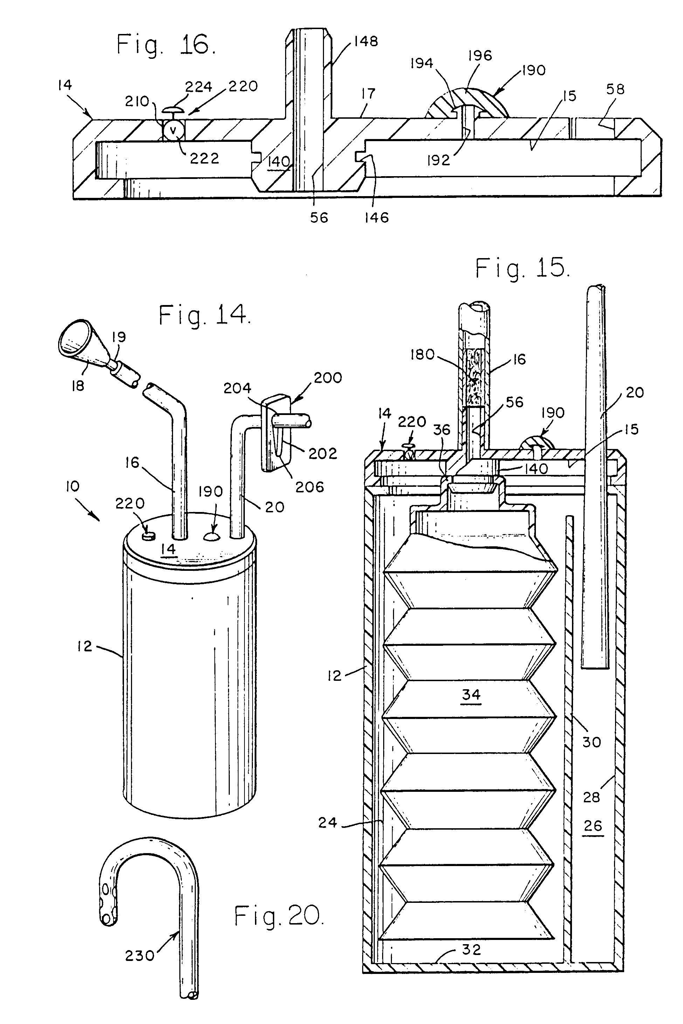

Several alternative structures for the joining of the bellows cap 36, cap 14 and suction tube 16 is illustrated in FIGURES 15 through 19. In one alternative arrangement, the bellows cap 36 is integrally formed with the inside of cap 14 and aperture 42 in bellows cap 36 is aligned directly with suction tube aperture 56 in cap 14. Referring to FIGURES 15 and 16, one way in which bellows cap 36 may be integrally formed with cap 14 is to have joining member 140 molded into the bottom of cap 14, which joining member 140 includes slot 146 to accommodate aperture 42 of bellows cap 36. Bellows cap 36 is permanently affixed to joining member 140 at the location of slot 146. Joining member 146 may also include an integrally formed nipple 148 which includes suction tube aperture 56 of cap 14 and rises above the top 17 of cap 14 and onto which the suction tube 16 may be placed. The joining member 146 includes a central orifice 56 from suction tube aperture 56 which also extends through the nipple 148, if present. Accordingly, the suction tube 16 rests only on nipple 148 and the flow of air passes from suction tube 16 through aperture 56 into bellows 34 which is attached to bellows cap 36.

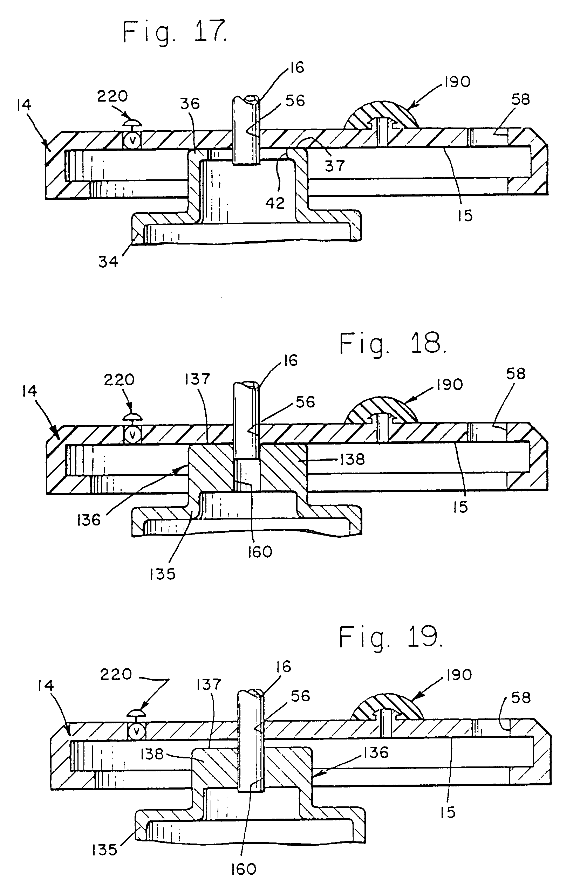

Alternatively, the joining member 140 can be eliminated and the top 37 of bellows cap 36 can be affixed directly to the underside 15 of cap 14 such that aperture 42 is aligned with suction tube aperture 56 in cap 14. Suction tube 16 extends through aperture 56 and directly into the bellows cap 36 and bellows 34. This is illustrated in FIGURE 17.

In the embodiment in which the joining member is eliminated, the central cap is preferably formed with a more solid interior and only a narrow orifice such that the suction tube 16 extends through suction tube aperture 56 and is aligned with narrower orifice 160. This is illustrated in greater detailed in FIGURE 18 in which bellows cap 136 includes a solid interior 138 having central orifice 160 in alignment with suction tube 16. It is also possible for the suction tube 16 to extend into central orifice 160. The solid bellows cap 136 includes side wall 135 which extends directly into the bellows. Bellows cap 136 is attached through its top 137 to the underside 15 of cap 14.

In another variation of this structure, if the press fit between suction tube 16 and the central orifice 160 is very tight, it is possible to have bellows cap 136 be supported directly by the suction tube 16 and the top 137 of bellows cap 136 need not be affixed to the underside 15 of cap 14. This is illustrated in FIGURE 19. In this embodiment, suction tube 16 extends well into orifice 160, as illustrated in FIGURE 19.

As best illustrated in FIGURES 2, 13, one end of patient tube 20 is disposed downward within mucus trap 26 of aspirator 10, at a distance in the range of about 3 to about 5 centimeters, or about one-third to about three-fourths of the length of the container, thereby insuring that mucus sucked into aspirator 10 falls into mucus trap 26, as long as aspirator 10 is in virtually any orientation other than upside down. Tapered portion 60 of patient tube 20 disposed within container 10 is uniformly tapered along the length intended to remain within aspirator 10, such that the diameter of tapered portion 60 of patient tube 20 is greater than the diameter of patient tube aperture 58.

Operation of aspirator 10 is clearly illustrated in FIGURES 2 and 3. Referring to FIGURE 2, bellows 34 is shown in its relaxed state. When a user sucks on mouthpiece 18, bellows 34 is contracted by air pressure conducted through patient tube 20, into the interior of aspirator 10, through mucus trap 26, over internal upstanding partition 30, and into vacuum creating chamber 24, allowing bellows 34 to contract. Naturally, as bellows 34 contracts, air is drawn into aspirator 10. When Murphy tip 22 of patient tube 20 is lodged near or in mucus or other bodily fluids, these liquids and other fluids are drawn into mucus trap 26. Liquid falls to the bottom of mucus trap 26, while gas flows into vacuum creating chamber 24. Referring to FIGURE 6, bellows 34 is illustrated in its contracted, or vacuum creating, state.

Naturally, when the medical care provider releases the sucking action from the mouthpiece 18, bellows 34 expands to its original position, as illustrated in FIGURE 2 and FIGURE 15, due to the memory of the silicon rubber material the bellows is made from. Expansion of bellows 34 naturally expels air from vacuum creating chamber 24, which can only be exhausted from aspirator 10 through patient tube 20. It has been found in practice that one suction cycle is usually sufficient to withdraw mucus and other liquids form the pertinent body cavity. If, however, more than one suction cycle is required, it has been found that allowing the air to be blown out through patient tube 20 into the body cavity being drained does not create any problems or difficulties. Hence, there is no real need for any type of valves that would prevent air from being blown out through patient tube 20.

Referring to FIGURE 4, there is shown a plan view of aspirator 10 according to the present invention. Such an aspirator may include a straight line internal upstanding partition 30, as illustrated in FIGURES 2 and 3, or it may include an arcuate internal upstanding partition 62, as illustrated in FIGURE 5, which better utilizes interior volume of aspirator 10, by allowing partition 62 to be closer to bellows 34, thereby allowing use of a smaller diameter cylindrical container 12.

Several enhancements for providing safer and more efficient operation are shown in FIGURES 14, 15 and 16. One such enhancement for safer utilization of the present invention is to provide a moisture trap 180 inside the suction tube 16 which acts as a moisture block. This is illustrated in FIGURE 15. The moisture trap 180 is a filter which permits air to pass when it is dry but if moisture is present, the filter swells up and occludes the passageway in suction tube 16, preventing air from passing. This is a valuable safety enhancement in the event the bellows or other sealing arrangement should break and moisture from the sucked contents of the baby's stomach enter the suction system. By providing this occlusion, the possibility of the the person working the apparatus sucking in such material or sucking in contaminated air is significantly reduced.

Referring to FIGURES 14 through 16, a further enhancement for more efficient operation of the present invention is the inclusion of an injection port 190 located in the area where mucus is collected, for example in the cap 14 immediately above the mucus trap 26 area of the present invention aspirator. The injection port 190 includes an opening 192 in the cap 14, and having a structural support member 194 extending through the opening 192 and which supports a diaphragm 196. After the mucus is collected, instead of opening the cap 14 to remove the mucus, the laboratory technician can place the needle of a syringe through the membrane 196, through opening 192 and into the mucus trap 26 area and draw out the mucus into the syringe, thereby eliminating the possibility of coming in direct conduct with contaminated mucus.

Referring to FIGURE 14, an additional enhancement is the inclusion of a clip 200 which includes an internal tapered opening 202. The clip 200 is placed around the patient tube 20 in an area between the cap 14 and the tip 22. During operation, the wider portion 204 of opening 202 surrounds the patient tube 20 so that the patient tube 20 is unobstructed. After the mucus has been sucked out fo the baby and is in the mucus trap 26, the clip 200 is slid so that its narrower portion 206 surrounds and pinches in the patient tube 20, to thereby occlude flow of liquid after the suction process is completed, thereby further assuring that no mucus can flow out of the patient tube and back into the patient.

The invention may also include means for releasing air from the container during the relaxation cycle of the bellows, that is, means for permitting air to be exhausted from the container while the bellows expand without having the displaced air exhausted through the patient tube. Referring to FIGURES 14 through 16, this air releasing means 220 may be a spring loaded valve 222 fit inside an aperture 210 in the top 14 (or it may be in the sidewall 12) of the container. The valve 222 is normally closed so no air can enter the container or escape from the container through this aperture 210. When a push button 224 or similar release mechanism connected to the valve 222 is activated, the valve 222 opens to let air escape from the chamber, thereby depressurizing the entire container so the bellows 34 can expand to its equilibrium position much more rapidly without the possibility of air or liquid being pushed up the patient tube 20 and back into the patient's stomach.

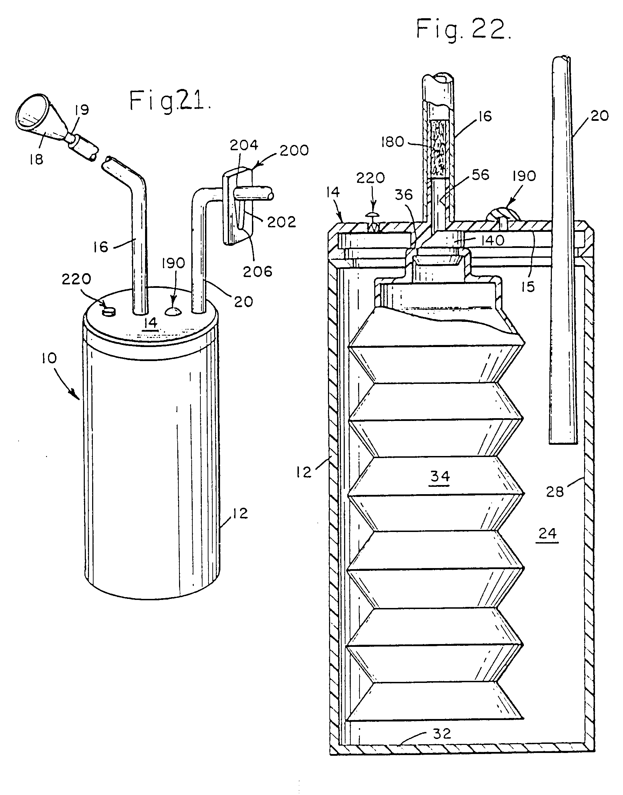

Referring to FIGURE 20, an additional improvement in the present invention is to include a Yankower suction tip 230 at the end of the patient tube so that the apparatus may be switched from patient to patient by replacement of the Yankower tip 230. In this way, the entire apparatus can remain in use and only the Yankower tip 230 changed when a new patient is worked on. This is especially useful in battlefield conditions where the present invention aspirator may be used to remove mucus and other debris from the mouth of a wounded soldier. Due to the extreme conditions of battle, it is easier to maintain the same apparatus and merely switch the patient tube tip when treating many different wounded soldiers.

Referring to FIGURE 8, there is shown an alternative embodiment of aspirator 11 having separate vacuum creating chamber 70, including suction tube aperture 72, and mucus trap 74, including patient tube aperture 76. Vacuum creating chamber 70 having cap 71 and mucus trap 74 having cap 75 are joined by sidewalls 78, 80, which are both tangent to the cross-sectional circles of vacuum creating chamber 70 and mucus trap 74. Internal upstanding partition 82 isolates mucus and other bodily liquids in mucus trap 74 from contact with vacuum creating chamber 70, but does not reach to the top of aspirator 11, as was described in conjunction with FIGURES 1 through 7 above. Sidewalls 78, 80 include frangible score line 84, allowing aspirator 11 to be cleanly and easily broken along frangible score line 84, thereby separating mucus trap 74 from vacuum creating chamber 70, providing a smaller container for use in analysis of the mucus or other bodily liquid trapped in mucus trap 74.

Referring to FIGURES 10 and 11, there is shown another alternative embodiment of aspirator 10 according to the present invention, in which vacuum creating chamber 70 is a cylindrical container wholly separate from mucus trap 74, which are joined together by rubber band 86, or other suitable fastener. Tube 94 connects vacuum chamber 70 and mucus trap 74. Tube 94 is disposed outside aspirator 13 across the top of aspirator 13, which includes a separate vacuum chamber cap 88, and a separate mucus trap cap 98, each of which may be removable. Tube 94 may be inserted into aperture 91 of cap 88, and aperture 93 of cap 98. Alternatively, of course, nipples may be integrally formed in caps 88, 98 to receive tube 94.

A suction tube (not shown) may be attached at suction tube aperture 89 by any convenient means, such as those described above. A patient tube may be conveniently attached as described with reference to FIGURES 1-7 through patient tube aperture 90. In the embodiment illustrated in FIGURES 10, 11, vacuum chamber cap 88 is not removable, but it may be a removable cap like that of FIGURE 7 if desired.

The embodiment illustrated in FIGURES 10, 11 allows easy and quick separation of vacuum creating chamber 70 from mucus trap 74, by removing rubber band 86 and removing the tube 94 from aperture 93. Mucus trap 74 can then be sealed by inserting a small stopper into each aperture of cap 98, and sent to the laboratory, where the contents can be analyzed to determine the cause of any medical problems the patient may be suffering. Mucus trap 74 of this embodiment has a separate mucus trap cap 98, having the depending circumferential skirt and inwardly projecting lip which allows mucus trap cap 98 to be removed from mucus trap 74 easily, as illustrated in FIGURE 7. In other respects, especially regarding the bellows vacuum and fluid separation system described above, the embodiment of FIGURES 10, 11 operates the same as that shown and described in reference to FIGURES 1-7.

The embodiment illustrated in FIGURE 10 is particularly preferred because it allows re-use of vacuum creating chamber 70 and bellows 34, which are the most expensive components of the system, while at the same time maintaining the separation of patient fluids from the medical care provider. If desired, aspirator 10 can be sterilized, particularly vacuum creating chamber 70, which will prevent spread of infection form one patient to another if the device is re-used.

In its preferred embodiment, however, aspirator 10 is disposable, and furthermore is sterile when shipped and is protected by sterile wrapping, thus insuring that bacteria or germs cultured from mucus trapped in mucus trap 74 is from the patient.

Referring to FIGURE 12, there is illustrated suction tube 16, including flared mouthpiece 18 having nipple 19 for insertion into tube 16. Referring to FIGURE 13, there is illustrated patient tube 20, including Murphy tip 22 having aperture 21, and tapered portion 60 for insertion upwardly through cap 14, or other cap or closure on mucus trap 26, 74, and so forth.

Referring to FIGURES 21 and 22, there is shown an alternative embodiment of the aspirator which is indentical to the aspirator shown in Figures 14 and 15, with the internal upstanding partition 30 eliminated. While this simplifies the structure, the mucus can now come in contact with the bellows.

Of course, the present invention is not intended to be restricted to any particular form or arrangement, or any specific embodiment disclosed herein, or any specific use, since the present invention may be modified in various particulars or relations without departing from the scope of the claimed invention shown and described herein, of which the apparatus shown are intended only for illustration and for disclosure of operative embodiments and not to show all of the various forms or modifications which might embody the invention.

The invention has been described in considerable detail in order to comply with the patent laws by providing a full public disclosure of one of its forms. Such detailed description is not, however, intended in any way to limit the broad features or principles of the invention, or the scope of the patent property to be granted.

## Claims

1. An aspirator for removing bodily fluids through human suction and subsequent collection of the removed bodily fluids, comprising a container (12) having a bottom wall (32); a side wall (28); a top (14) having a first aperture (58) therein and a second aperture (56) therein spaced apart from the first aperture, the top being removably attached to said container; a first hollow tube (20) having two ends, with the first end (60) inserted through said first aperture and the second end having at least one opening (21) adjacent its tip (22); and a second hollow tube in third communication with said second aperture; characterized by
(a) an upstanding partition (30) within said container to divide the container into a first chamber (26) and a second chamber (24);
(b) said upstanding partition extending from the bottom of said container to a distance between the bottom of said container and said top to create a division opening between said first and second chambers to thereby permit gaseous communication but not liquid communication between said first and second chambers;
(c) said first aperture opening into said first chamber and said second aperture opening into said second chamber;
(d) said first hollow tube extending for a distance below said division opening (20) and into said first chamber for a total distance inside the container in the range from about one-third to about three-fourths of the length of the container and said second end extends for a distance beyond said top;
(e) a mating member (40) having a central longitudinal opening extending through said second aperture such that a first end (48) of the mating member protrudes through and above said top and the second end (44) of the mating member extends into the container above said second chamber;
(f) the first end of said second hollow tube (16) being attached to said first end of the mating member and the second end of said second hollow tube being attached to a mouthpiece (18);
(g) a flexible bellows member (34) having an integrally formed flexible bellows and terminating in a sealed bottom (38) at one end and affixed to a bellows cap (36) at its other end; and
(h) said bellows cap further having an opening (42) through which it is attached to the second end (41) of said mating member (40) to thereby provide a closed system permitting gaseous communication therethrough between the mouthpiece, the second hollow tube, the mating member and the bellows, and to further permit the flexible bellows to extend into said second chamber for a distance beyond said division opening such that the flexible bellows is in its fully expanded position when in its equilibrium state;
(i) whereby in use, the second end of said first hollow tube is inserted into the patient from which bodily fluid is to be removed and the mouthpiece of the second hollow tube is sucked on by the person treating the patient, and suction through the mouthpiece will cause said flexible bellows to contract to thereby create a vacuum in said second chamber which through gaseous communication with said first chamber causes air and bodily fluids from the patient to be sucked into and remain in the first chamber while the airtight system from the mouthpiece through the flexible bellows prevents any direct communication of air or bodily fluids between the patient and the person treating the patient.

2. An aspirator as claimed in Claim 1 further characterized in that said upstanding partition further comprises a flat straight partition which is sealingly engaged with said bottom wall and along two lines of contact with said side wall of said container, and wherein said upstanding partition projects upward from said bottom wall a distance within the range of one-third to seven-eights of the length of said container.

3. An aspirator as claimed in Claim 1 further characterized in that said container is circular and said upstanding partition further comprises an arcuate partition (62) shaped in a concave formation facing said second chamber and conforming to the shape of said bellows to thereby permit close communication between said bellows and the sidewall of the container and upstanding partition to enhance the vacuum created when said bellows contracts due to suction applied to said mouthpiece.

4. An aspirator as claimed in Claim 3 further characterized in that said upstanding partition is sealingly engaged with said bottom wall and along two lines of contact with said side wall of said container, and wherein said upstanding partition projects upward from said bottom wall a distance within the range of one-third to seven-eights of the length of said container.

5. An aspirator as claimed in any one of the preceding claims further characterized in that the length of said first hollow tubing proximate its first end includes a tapered end portion (60) such that said first hollow tubing cannot be withdrawn upward through said container top.

6. An aspirator as claimed in Claim 1 further characterized in that said container is circular and said top further comprises a circular top having a circumferential depending skirt portion and an inwardly projecting circumferential lip (54) attached to the bottom of said skirt.

7. An aspirator as claimed in any one of the preceding claims further characterized in that said bellows comprises a unitary cylindrical bellows made of an airtight elastic membrane.

8. An aspirator as claimed in any one of the preceding claims further characterized by means (220) for permitting air to be exhausted from the container after the sucking action has ceased and while the bellows expand to its equilibrium state without having the displaced air exhausted through the first hollow tube.

9. An aspirator as claimed in any one of the preceding claims wherein the second end of said first hollow tube terminates in a Murphy tip (22).

10. An aspirator for removing bodily fluids through human suction and subsequent collection of the removed bodily fluids, comprising a container (12) having a bottom wall (32); and a side wall (28); a top (14) having a first aperture (58) therein and a second aperture (56) therein spaced apart from the first aperture, the top being removably attached to said container; a first hollow tube (20) having two ends, with the first end (60) inserted through said first aperture in the top and the second end having at least one opening (21) adjacent its tip (22); and a second hollow tube (16) in fluid communication with said second aperture; and characterized by
(a) an upstanding partition (30) within said container to divide the container into a first chamber (26) and a second chamber (24);
(b) said upstanding partition extending from the bottom of said container to a distance between the bottom of said container and said top to create a division opening between said first and second chambers to thereby permit gaseous communication but not liquid communication between said first and second chambers;
(c) said first aperture opening into said first chamber and said second aperture opening into said second chamber;
(d) the first end of the first hollow tube extending for a distance below said division opening and into said first chamber and said second end extends for a distance beyond said top;
(e) a flexible bellows member (34) having an integrally formed flexible bellows and terminating in a sealed bottom (38) at one end and affixed to a bellows cap (36) having an opening (160) at its other end; and
(f) the first end of said second hollow tube (16) inserted through said second aperture in the top and further inserted through and retained by the opening (160) in said bellows cap (34) and the second end of the second hollow tube extending for a distance beyond said top and attached at its tip to a mouthpiece (18), to thereby provide a closed system permitting gaseous communication therethrough between the mouthpiece, the second hollow tube, and the bellows, and to further permit the flexible bellows to extend into said second chamber for a distance beyond said division opening such that the flexible bellows is in its fully expanded position when in its equilibrium state;
(g) whereby in use, the second end of said first hollow tube is inserted into the patient from which bodily fluid is to be removed and the mouthpiece of the second hollow tube is sucked on by the person treating the patient, and suction through the mouthpiece will cause said flexible bellows to contract to thereby create a vacuum in said second chamber which through gaseous communication with said first chamber causes air and bodily fluids from the patient to be sucked into and remain in the first chamber while the airtight system from the mouthpiece through the flexible bellows prevents any direct communication of air or bodily fluids between the patient and the person treating the patient.

11. An aspirator as claimed in Claim 10 further characterized in that said first hollow tube extends through said first aperture and into said first chamber for a total distance inside the container in the range from about one-third to about three-fourths of the length of the container.

12. An aspirator as claimed in Claims 10 or 11 further characterized in that said upstanding partition is a flat straight partition which is sealingly engaged with said bottom wall and along two lines of contact with said side wall of said container, and wherein said upstanding partition projects upward from said bottom wall a distance within the range of one-third to seven-eights of the length of said container.

13. An aspirator as claimed in Claim 10 further characterized in that said container is circular and said upstanding partition further comprises an arcuate partition (62) shaped in a concave formation facing said second chamber and conforming to the shape of said bellows to thereby permit close communication between said bellows and the sidewall of the container and upstanding partition to enhance the vacuum created when said bellows contracts due to suction applied to said mouthpiece.

14. An aspirator as claimed in Claim 13 further characterized in that said upstanding partition is sealingly engaged with said bottom wall and along two lines of contact with said side wall of said container, and wherein said upstanding partition projects upward from said bottom wall a distance within the range of one-third to seven-eights of the length of said container.

15. An aspirator as claimed in any one of Claims 10-14 further characterized in that the length of said first hollow tubing proximate its first end includes a tapered end portion (60) such that said first hollow tubing cannot be withdrawn upward through said container top.

16. An aspirator as claimed in any one of Claims 10-15 further characterized in that said container is circular and said top further comprises a circular top having a circumferential depending skirt portion and an inwardly projecting circumferential lip (54) attached to the bottom of said skirt.

17. An aspirator as claimed in any one of Claims 10-16 further characterized in that said bellows comprises a unitary cylindrical bellows made of an airtight elastic membrane.

18. An aspirator as claimed in any one of Claims 10-17 further characterized by means (220) for permitting air to be exhausted from the container after the sucking action has ceased and while the bellows expand to its equilibrium state without having the displaced air exhausted through the first hollow tube.

19. An aspirator as claimed in any one of Claims 10-18 wherein the second end of said first hollow tube terminates in a Murphy tip (22).

## Patentansprüche

1. Aspirator zur Entfernung von Körperflüssigkeiten durch Absaugen durch den Menschen und nachfolgendem Sammeln der entfernten Körperflüssigkeiten, umfassend: einen Behälter (12) mit einem Boden (32), einer Seitenwand (28), einem Oberteil (14), das darin eine erste Öffnung (58) und darin eine von der ersten Öffnung beabstandete zweite Öffnung (56) aufweist, wobei das Oberteil an dem Behälter entfernbar angebracht ist; ein erstes Hohlrohr (20) mit zwei Enden, wobei das erste Ende (60) durch die erste Öffnung eingeführt wird und das zweite Ende mindestens eine Öffnung (21) neben seiner Spitze (22) hat; und ein in dritter Verbindung mit der zweiten Öffnung befindliches zweites Hohlrohr,
dadurch gekennzeichnet, daß
(a) eine aufrechtstehende Trennwand (30) im Inneren des Behälters den Behälter in eine erste Kammer (26) und eine zweite Kammer (24) unterteilt,
(b) sich die aufrechtstehende Trennwand vom Boden des Behälters bis zu einer Distanz zwischen dem Boden des Behälters und dem Oberteil erstreckt, so daß eine Trennungsöffnung zwischen der ersten und zweiten Kammer geschaffen wird, um dadurch zwischen der ersten und zweiten Kammer eine Gasverbindung, nicht aber eine Flüssigkeitsverbindung zu ermöglichen,
(c) die erste Öffnung sich in die erste Kammer öffnet und die zweite Öffnung sich in die zweite Kammer öffnet,
(d) das erste Hohlrohr (20) sich bis zu einer Distanz unterhalb der Trennungsöffnung und in die erste Kammer für eine Gesamtdistanz im Inneren des Behälters im Bereich von etwa einem Drittel bis zu etwa drei Viertel der Länge des Behälters erstreckt und das zweite Ende um eine Distanz über das Oberteil hinausragt,
(e) ein Paßteil mit einer zentralen Öffnung in Längsrichtung, das sich derart durch die zweite Öffnung erstreckt, daß ein erstes Ende (48) des Paßteils durch und über das Oberteil hinausragt und sich das zweite Ende (44) des Paßteils in den Behälter oberhalb der zweiten Kammer erstreckt,
(f) das erste Ende des zweiten Hohlrohres (16) an dem ersten Ende des Paßteils angebracht und das zweite Ende des zweiten Hohlrohres an einem Mundstück angebracht (18) ist,
(g) ein flexibles Balgteil (34) einen einstückigen flexiblen Balg hat und an dem einen Ende mit einem dicht verschlossenen Boden (38) abschließt und mit seinem anderen Ende an einer Balgkappe (36) befestigt ist,
(h) die Balgkappe ferner eine Öffnung (42) hat, durch die hindurch sie an dem zweiten Ende (41) des Paßteils (40) befestigt ist, um dadurch ein geschlossenes System zu schaffen, durch welches System hindurch zwischen dem Mundstück, dem zweiten Hohlrohr, dem Paßteil und dem Balg eine Gasverbindung ermöglicht wird, und um ferner dem flexiblen Balg zu ermöglichen, sich über eine Distanz in die zweite Kammer derart über die Trennungsöffnung hinaus zu erstrecken, daß der flexible Balg in seiner voll ausgezogenen Stellung ist, wenn er sich im Gleichgewichtszustand befindet,
(i) in Gebrauch das zweite Ende des ersten Hohlrohres in den Patienten eingeführt wird, von dem Körperflüssigkeit entfernt werden soll, und das Mundstück des zweiten Hohlrohres von der Person angesaugt wird, die den Patienten behandelt und das Saugen durch dieses Mundstück bewirkt, daß sich der flexible Balg zusammenzieht und dadurch in der zweiten Kammer einen Unterdruck schafft, welcher durch die Gasverbindung mit der ersten Kammer bewirkt, daß Luft und Körperflüssigkeiten von dem Patienten in die erste Kammer gesaugt werden und dort verbleiben, während das luftdichte System von dem Mundstück durch den flexiblen Balg jede direkte Verbindung von Luft oder Körperflüssigkeiten zwischen dem Patienten und der den Patienten behandelnden Person verhindert.

2. Aspirator nach Anspruch 1, ferner dadurch gekennzeichnet, daß die aufrechtstehende Trennwand ferner umfaßt: eine ebene, gerade Trennwand, die sich in einem dichtenden Eingriff mit dem Boden und entlang zweier Berührungslinien mit der Seitenwand des Behälters befindet, wobei sich die aufrechtstehende Trennwand von dem Boden um eine Distanz im Bereich von einem Drittel bis zu sieben Achtel der Länge des Behälters nach oben erstreckt.

3. Aspirator nach Anspruch 1, ferner dadurch gekennzeichnet, daß der Behälter kreisrund ist und die aufrechtstehende Trennwand ferner umfaßt: eine gegenüber dieser zweiten Kammer befindliche, bogenförmige und konkav entsprechend dem Balg ausgebildete Trennwand (62), um dadurch eine enge Verbindung mit dem Balg zu ermöglichen, um den erzeugten Unterdruck zu verstärken, wenn sich durch Aufbringen eines Sogs an dem Mundstück der Balg zusammenzieht.

4. Aspirator nach Anspruch 3, ferner dadurch gekennzeichnet, daß sich die aufrechtstehende Trennwand in einem dichtenden Eingriff mit dem Boden und entlang zweier Berührungslinien mit der Seitenwand des Behälters befindet, wobei sich die aufrechtstehende Trennwand von dem Boden um eine Distanz im Bereich von einem Drittel bis zu sieben Achtel der Länge des Behälters nach oben erstreckt.

5. Aspirator nach einem der vorstehenden Ansprüche, ferner dadurch gekennzeichnet, daß die Länge des ersten Hohlrohres in der Nähe seines ersten Endes einen solchen konischen Endabschnitt (60) aufweist, daß die Länge das erste Hohlrohr durch das Oberteil des Behälters nicht nach oben herausgezogen werden kann.

6. Aspirator nach Anspruch 3, ferner dadurch gekennzeichnet, daß der Behälter kreisrund ist und das Oberteil ferner umfaßt: ein kreisrundes Oberteil mit einem am Umfang nach unten ragenden Randabschnitt und einer nach innen stehenden, am Umfang befindlichen Kante (54), die an der Unterseite des Randes angebracht ist.

7. Aspirator nach einem der vorstehenden Ansprüche, ferner dadurch gekennzeichnet, daß der Balg einen einstückigen zylindrischen Balg umfaßt, der aus einer luftdichten elastischen Membran gefertigt ist.

8. Aspirator nach einem der vorstehenden Ansprüche, ferner gekennzeichnet durch eine Vorrichtung (220), mit der Luft aus dem Behälter abgelassen werden kann, nachdem das Absaugen eingestellt worden ist und während der Balg in sein Gleichgewichtszustand ausgezogen wird, ohne daß die verdrängte Luft durch das erste Hohlrohr ausgestoßen wurde.

9. Aspirator nach einem der vorstehenden Ansprüche, bei welchem das zweite Ende des ersten Hohlrohres in einer Murphy-Spitze endet (22).

10. Aspirator zur Entfernung von Körperflüssigkeiten durch Absaugen durch den Menschen und nachfolgendem Sammeln der entfernten Körperflüssigkeiten, umfassend: einen Behälter (12) mit einem Boden (32), einer Seitenwand (28), einem Oberteil (14), das darin eine erste Öffnung (58) und darin eine von der ersten Öffnung beabstandete zweite Öffnung (56) aufweist, wobei das Oberteil an dem Behälter entfernbar angebracht ist; ein erstes Hohlrohr (20) mit zwei Enden, wobei das erste Ende (60) durch die erste Öffnung in das Oberteil eingeführt wird und das zweite Ende mindestens eine Öffnung (21) neben seiner Spitze (22) hat; und ein zweites Hohlrohr (16) in Flüssigkeitsverbindung mit der zweiten Öffnung steht, dadurch gekennzeichnet, daß
(a) eine aufrechtstehende Trennwand (30) im Inneren des Behälters den Behälter in eine erste Kammer (26) und eine zweite Kammer (24) unterteilt,
(b) sich die aufrechtstehende Trennwand vom Boden des Behälters bis zu einer Distanz zwischen dem Boden des Behälters und dem Oberteil erstreckt, so daß eine Trennungsöffnung zwischen der ersten und zweiten Kammer geschaffen wird, um dadurch zwischen der ersten und zweiten Kammer eine Gasverbindung, nicht aber eine Flüssigkeitsverbindung zu ermöglichen,
(c) die erste Öffnung sich in die erste Kammer öffnet und die zweite Öffnung sich in die zweite Kammer öffnet,
(d) das erste Ende des Hohlrohres sich bis zu einer Distanz unterhalb der Trennungsöffnung und in die erste Kammer erstreckt und das zweite Ende um eine Distanz über das Oberteil hinausragt,
(e) ein flexibles Balgteil (34) einen einstückigen flexiblen Balg hat und an dem einen Ende mit einem dicht verschlossenen Boden (38) abschließt und mit seinem anderen Ende an einer Balgkappe (36) mit einer Öffnung (160) befestigt ist,
(f) das erste Ende des zweiten Hohlrohres (16) durch die zweite Öffnung in dem Oberteil und ferner durch die Öffnung (160) in der Balgkappe (34) eingesetzt und von dieser gehalten wird, und das zweite Ende des zweiten Hohlrohres sich um eine Distanz über das Oberteil hinaus erstreckt und an seiner Spitze ein Mundstück (18) angebracht hat, um dadurch ein geschlossenes System zu schaffen, durch welches System hindurch zwischen dem Mundstück, dem zweiten Hohlrohr und dem Balg eine Gasverbindung ermöglicht wird, und um ferner dem flexiblen Balg zu ermöglichen, sich über eine Distanz in die zweite Kammer derart über die Trennungsöffnung hinaus zu erstrecken, daß der flexible Balg in seiner voll ausgezogenen Stellung ist, wenn er sich im Gleichgewichtszustand befindet,
(g) in Gebrauch das zweite Ende des ersten Hohlrohres in den Patienten eingeführt wird, von dem Körperflüssigkeit entfernt werden soll, und das Mundstück des zweiten Hohlrohres von der Person angesaugt wird, die den Patienten behandelt und das Saugen durch dieses Mundstück bewirkt, daß sich der flexible Balg zusammenzieht und dadurch in der zweiten Kammer einen Unterdruck schafft, welcher durch die Gasverbindung mit der ersten Kammer bewirkt, daß Luft und Körperflüssigkeiten von dem Patienten in die erste Kammer gesaugt werden und dort verbleiben, während das luftdichte System von dem Mundstück durch den flexiblen Balg jede direkte Verbindung von Luft oder Körperflüssigkeiten zwischen dem Patienten und der den Patienten behandelnden Person verhindert.

11. Aspirator nach Anspruch 10, ferner dadurch gekennzeichnet, daß sich das erste Hohlrohr durch die erste Öffnung und in die erste Kammer für eine Gesamtdistanz im Inneren des Behälters im Bereich von etwa einem Drittel bis zu etwa drei Viertel der Länge des Behälters erstreckt .

12. Aspirator nach Anspruch 10 oder 11, ferner dadurch gekennzeichnet, daß die aufrechtstehende Trennwand ferner umfaßt: eine ebene, gerade Trennwand, die sich in einem dichtenden Eingriff mit dem Boden und entlang zweier Berührungslinien mit der Seitenwand des Behälters befindet, wobei sich die aufrechtstehende Trennwand von dem Boden um eine Distanz im Bereich von einem Drittel bis zu sieben Achtel der Länge des Behälters nach oben erstreckt.

13. Aspirator nach Anspruch 10, ferner dadurch gekennzeichnet, daß der Behälter kreisrund ist und die aufrechtstehende Trennwand ferner umfaßt: eine gegenüber dieser zweiten Kammer befindliche, bogenförmige und konkav entsprechend dem Balg ausgebildete Trennwand (62), um dadurch eine enge Verbindung mit dem Balg zu ermöglichen, um den erzeugten Unterdruck zu verstärken, wenn sich durch Aufbringen eines Sogs an dem Mundstück der Balg zusammenzieht.

14. Aspirator nach Anspruch 13, ferner dadurch gekennzeichnet, daß sich die aufrechtstehende Trennwand in einem dichtenden Eingriff mit dem Boden und entlang zweier Berührungslinien mit der Seitenwand des Behälters befindet, wobei sich die aufrechtstehende Trennwand von dem Boden um eine Distanz im Bereich von einem Drittel bis zu sieben Achtel der Länge des Behälters nach oben erstreckt.

15. Aspirator nach einem der Ansprüche 10 bis 14, ferner dadurch gekennzeichnet, daß die Länge des ersten Hohlrohres in der Nähe seines ersten Endes einen solchen konischen Endabschnitt (60) aufweist, daß die Länge das erste Hohlrohr durch das Oberteil des Behälters nicht nach oben herausgezogen werden kann.

16. Aspirator nach einem der Ansprüche 10 bis 15, ferner dadurch gekennzeichnet, daß der Behälter kreisrund ist und das Oberteil ferner umfaßt: ein kreisrundes Oberteil mit einem am Umfang nach unten ragenden Randabschnitt und einer nach innen stehenen, am Umfang befindlichen Kante (54), die an der Unterseite des Randes angebracht ist.

17. Aspirator nach einem der vorstehenden Ansprüche 10 bis 16, ferner dadurch gekennzeichnet, daß der Balg einen einstückigen zylindrischen Balg umfaßt, der aus einer luftdichten elastischen Membran gefertigt ist.

18. Aspirator nach einem der vorstehenden Ansprüche 10 bis 17, ferner gekennzeichnet durch eine Vorrichtung (220), mit der Luft aus dem Behälter abgelassen werden kann, nachdem das Absaugen eingestellt worden ist und während der Balg in sein Gleichgewichtszustand ausgezogen wird, ohne daß die verdrängte Luft durch das erste Hohlrohr ausgestoßen wurde.

19. Aspirator nach einem der vorstehenden Ansprüche 10 bis 18, bei welchem das zweite Ende des ersten Hohlrohres in einer Murphy-Spitze endet (22).

## Revendications

1. Aspirateur pour retirer les fluides corporels par aspiration humaine puis collecte des fluides corporels retirés, comprenant un récipient (12) comportant une paroi inférieure (32), une paroi latérale (28), un sommet (14) comportant une première ouverture (58) et une seconde ouverture (56) distante de la première ouverture, le sommet étant fixé de manière amovible sur ledit récipient, un premier tube creux (20) comportant deux extrémités, la première extrémité (60) étant insérée par ladite première ouverture et la seconde extrémité présentant au moins une ouverture (21) adjacente à son extrémité (22), et un second tube creux en troisième communication avec ladite seconde ouverture, caractérisé par
(a) une séparation verticale (30) à l'intérieur dudit récipient pour diviser ledit récipient en une première chambre (26) et en une seconde chambre (24);
(b) le fait que ladite séparation verticale s'étend depuis le fond dudit récipient jusqu'à une certaine distance entre le fond dudit récipient et ledit sommet pour créer une ouverture de division entre lesdites première et seconde chambres pour permettre ainsi une communication gazeuse mais pas de communication liquide entre lesdites première et seconde chambres ;
(c) le fait que ladite première ouverture débouche dans ladite première chambre et que ladite seconde ouverture débouche dans ladite seconde chambre ;
(d) le fait que ledit premier tube creux s'étend sur une certaine distance sous ladite ouverture de division (20) et dans ladite première chambre sur une distance totale à l'intérieur du récipient située dans la plage d'environ 1/3 à environ 3/4 de la longueur du récipient et que ladite seconde extrémité s'étend sur une certaine distance au-delà dudit sommet ;
(e) un élément de raccordement (40) comportant une ouverture longitudinale centrale qui s'étend à travers ladite seconde ouverture de manière qu'une première extrémité (48) de l'élément de raccordement fasse saillie à travers ledit sommet et au-dessus de celui-ci et que la seconde extrémité (44) de l'élément de raccordement s'étende dans le récipient au-dessus de ladite seconde chambre ;
(f) le fait que la première extrémité dudit second tube creux (16) est fixée à ladite première extrémité de l'élément de raccordement et que la seconde extrémité dudit second tube creux est fixée à un embout buccal (18) ;
(g) un élément à soufflet flexible (34) comportant un soufflet flexible formé d'une pièce et terminé par un fond étanche (38) à une extrémité, et fixé à un capuchon de soufflet (36) à son autre extrémité, et
(h) par le fait que ledit capuchon de soufflet comporte en outre une ouverture (42) par laquelle il est fixé à la seconde extrémité (41) dudit élément de raccordement (40) pour former ainsi un système fermé permettant une communication gazeuse entre l'embout buccal, le second tube creux, l'élément de raccordement et le soufflet, et pour permettre en outre au soufflet flexible de s'étendre dans ladite seconde chambre sur une distance au-delà de ladite ouverture de division telle que le soufflet flexible occupe sa position totalement expansée lorsqu'il est dans son état d'équilibre ;
(i) de sorte que, en utilisation, la seconde extrémité dudit premier tube creux est insérée dans le patient duquel un fluide corporel doit être retiré et la personne qui traite le patient exerce une aspiration sur l'embout buccal du second tube creux, et l'aspiration par l'intermédiaire de l'embout buccal amène ledit soufflet flexible à se contracter pour créer un vide dans ladite seconde chambre qui, par communication gazeuse avec ladite première chambre, amène l'air et les fluides corporels du patient à être aspirés dans la première chambre et à y rester tandis que le système étanche à l'air de l'embout buccal au soufflet flexible empêche toute communication directe de l'air ou des fluides corporels entre le patient et la personne qui traite le patient.

2. Aspirateur selon la revendication 1, caractérisé en outre en ce que ladite séparation verticale comprend également une séparation rectiligne plate qui est en contact étanche avec ladite paroi inférieure et suivant deux lignes de contact avec ladite paroi latérale dudit récipient, et dans lequel ladite séparation verticale fait saillie vers le haut depuis ladite paroi inférieure sur une distance comprise dans la plage de 1/3 à 7/8 de la longueur dudit récipient.

3. Aspirateur selon la revendication 1, caractérisé en outre en ce que ledit récipient est circulaire et ladite séparation verticale comprend en outre une séparation arquée (62) présentant une formation concave tournée vers ladite seconde chambre et épousant la forme dudit soufflet pour permettre ainsi une communication fermée entre ledit soufflet et la paroi latérale du récipient et une cloison verticale pour augmenter le vide créé lorsque ledit soufflet se contracte par suite de l'aspiration appliquée audit embout buccal.

4. Aspirateur selon la revendication 3, caractérisé en outre en ce que ladite séparation verticale est en contact étanche avec ladite paroi inférieure et suivant deux lignes de contact avec ladite paroi latérale dudit récipient, et dans lequel ladite séparation verticale fait saillie vers le haut depuis ladite paroi inférieure sur une distance située dans la plage de 1/3 à 7/8 de la longueur dudit récipient.

5. Aspirateur selon l'une quelconque des revendications précédentes, caractérisé en outre en ce que la longueur dudit premier tube creux à proximité de sa première extrémité comprend une partie terminale amincie (60) de sorte que le premier tube creux ne peut pas être retiré verticalement par ledit sommet du récipient.

6. Aspirateur selon la revendication 1, caractérisé en outre en ce que ledit récipient est circulaire et ledit sommet comprend en outre un sommet circulaire présentant une partie extrême dépendante circonférentielle et une lèvre circonférentielle (54) qui fait saillie vers l'intérieur et qui est fixée au fond de ladite partie extrême.

7. Aspirateur selon l'une quelconque des revendications précédentes, caractérisé en outre en ce que ledit soufflet comprend un soufflet cylindrique unitaire constitué par une membrane élastique étanche à l'air.

8. Aspirateur selon l'une quelconque des revendications précédentes, caractérisé en outre par un dispositif (220) permettant à l'air d'être évacué du récipient après la fin de l'action d'aspiration et tandis que le soufflet se détend jusqu'à son état d'équilibre sans que l'air déplacé soit évacué par le premier tube creux.

9. Aspirateur selon l'une quelconque des revendications précédentes, dans lequel la seconde extrémité dudit premier tube creux se termine par une extrémité de Murphy (22).

10. Aspirateur pour retirer les fluides corporels par aspiration humaine puis collecte des fluides corporels retirés, comprenant un récipient (12) comportant une paroi inférieure (32), une paroi latérale (28), un sommet (14) présentant une première ouverture (58) et une seconde ouverture (56) distance de la première ouverture, le sommet étant fixé de manière amovible sur ledit récipient, un premier tube creux (20) comportant deux extrémités, la première extrémité (60) étant insérée par ladite première ouverture dans le sommet et la seconde extrémité ayant au moins une ouverture (21) adjacente à son extrémité (22), et un second tube creux (16) en communication fluide avec ladite seconde ouverture, et caractérisé par
(a) une séparation verticale (30) dans ledit récipient pour diviser le récipient en une première chambre (26) et en une seconde chambre (24) ;
(b) le fait que ladite séparation verticale s'étend depuis le fond dudit récipient jusqu'à une certaine distance entre le fond dudit récipient et ledit sommet pour créer une ouverture de division entre lesdites première et seconde chambres pour permettre ainsi une communication gazeuse mais pas de communication liquide entre lesdites première et seconde chambres ;
(c) le fait que ladite première ouverture débouche dans ladite première chambre et que ladite seconde ouverture débouche dans ladite seconde chambre ;
(d) le fait que la première extrémité du premier tube creux s'étend sur une certaine distance au-dessous de ladite ouverture de division et dans ladite première chambre et que ladite seconde extrémité s'étend sur une certaine distance au-delà dudit sommet ;
(e) un élément à soufflet flexible (34) comportant un soufflet flexible formé d'une pièce et se terminant par un fond étanche (38) à une extrémité et fixé à un capuchon de soufflet (36) comportant une ouverture (160) à son autre extrémité ;
(f) le fait que la première extrémité dudit second tube creux (16) est insérée par ladite seconde ouverture dans le sommet et est insérée également dans et retenue par l'ouverture (160) dans ledit capuchon de soufflet (34) et que la seconde extrémité du second tube creux s'étend sur une certaine distance au-delà dudit sommet et est fixée à son extrémité à un embout buccal (18) pour former ainsi un système fermé permettant une communication gazeuse entre l'embout buccal, le second tube creux et le soufflet, et pour permettre en outre au soufflet flexible de s'étendre dans ladite seconde chambre sur une distance au-delà de ladite ouverture de division telle que le soufflet flexible se trouve dans sa position totalement expansée lorsqu'il est dans son état d'équilibre ;
(g) de sorte que, en utilisation, la seconde extrémité dudit premier tube creux est insérée dans le patient duquel un fluide corporel doit être retiré et la personne qui traite le patient exerce une aspiration sur l'embout buccal du second tube creux, et l'aspiration par l'embout buccal amène ledit soufflet creux à se contracter pour créer ainsi un vide dans ladite seconde chambre qui, par communication gazeuse avec ladite première chambre, amène l'air et les fluides corporels du patient à être aspirés dans ladite première chambre et à y rester tandis que le système étanche à l'air de l'embout buccal au soufflet flexible empêche toute communication directe de l'air ou des fluides corporels entre le patient et la personne qui traite le patient.

11. Aspirateur selon la revendication 10, caractérisé on outre en ce que ledit premier tube creux s'étend par ladite première ouverture et dans ladite première chambre sur une distance totale à l'intérieur du récipient comprise dans la plage d'environ 1/3 à environ 3/4 de la longueur du récipient.

12. Aspirateur selon la revendication 10 ou 11, caractérisé en outre en ce que ladite séparation verticale est une séparation rectiligne plate qui est en contact étanche avec ladite paroi inférieure et suivant deux lignes de contact avec ladite paroi latérale dudit récipient, et dans lequel ladite séparation verticale fait saillie vers le haut depuis ladite paroi inférieure sur une distance comprise dans la plage de 1/3 à 7/8 de la longueur dudit récipient.

13. Aspirateur selon la revendication 10, caractérisé en outre en ce que ledit récipient est circulaire et ladite séparation verticale comprend en outre une séparation arquée (62) présentant une formation concave tournée vers ladite seconde chambre et épousant la forme dudit soufflet pour permettre ainsi une communication fermée entre ledit soufflet et la paroi latérale du récipient et une séparation verticale pour augmenter le vide créé lorsque ledit soufflet se contracte par suite de l'aspiration appliquée audit embout buccal.

14. Aspirateur selon la revendication 13, caractérisé en outre en ce que ladite séparation verticale est en contact étanche avec ladite paroi inférieure et suivant deux lignes de contact avec ladite paroi latérale dudit récipient, et dans lequel ladite séparation verticale fait saillie vers le haut depuis ladite paroi inférieure sur une distance comprise dans la plage de 1/3 à 7/8 de la longueur dudit récipient.

15. Aspirateur selon l'une quelconque des revendications 10 à 14, caractérisé en outre en ce que la longueur dudit premier tube creux à proximité de sa première extrémité comprend une partie extrême amincie (60) de sorte que ledit premier tube creux ne peut pas être retiré verticalement par ledit sommet du récipient.

16. Aspirateur selon l'une quelconque des revendications 10 à 15, caractérisé en outre en ce que ledit récipient est circulaire et ledit sommet comprend en outre un sommet circulaire comportant une partie extrême dépendante circonférentielle et une lèvre circonférentielle (54) qui fait saillie vers l'intérieur et qui est fixée au fond de ladite partie extrême.

17. Aspirateur selon l'une quelconque des revendications 10 à 16, caractérisé en outre en ce que ledit soufflet comprend un soufflet cylindrique unitaire constitué par une membrane élastique étanche à l'air.

18. Aspirateur selon l'une quelconque des revendications 10 à 17, caractérisé en outre par un dispositif (220) pour permettre à l'air d'être évacué du récipient après la fin de l'action d'aspiration et tandis que le soufflet se détend pour atteindre son état d'équilibre sans que l'air déplacé ne soit évacué par le premier tube creux.

19. Aspirateur selon l'une quelconque des revendications 10 à 18, dans lequel la seconde extrémité dudit premier tube creux se termine par une extrémité de Murphy (22).
